(19) **Europäisches Patentamt / European Patent Office / Office européen des brevets**

(11) **EP 2 343 008 A1**

(12) **EUROPEAN PATENT APPLICATION**
published in accordance with Art. 153(4) EPC

(43) Date of publication:
**13.07.2011 Bulletin 2011/28**

(51) Int Cl.:
**A61B 5/021** (2006.01)

(21) Application number: **09820286.4**

(22) Date of filing: **06.02.2009**

(86) International application number:
**PCT/ES2009/000064**

(87) International publication number:
**WO 2010/043728 (22.04.2010 Gazette 2010/16)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **16.10.2008 ES 200802916**

(71) Applicant: **Sabirmedical, S.L.**
**08028 Barcelona (ES)**

(72) Inventors:
• **RIBAS RIPOLL, Vicente Jorge**
  **E-08028 Barcelona (ES)**
• **GARCÍA LLORENTE, Víctor Manuel**
  **E-08028 Barcelona (ES)**
• **MONTE MORENO, Enrique**
  **E-08034 Barcelona (ES)**

(74) Representative: **ZBM Patents**
**Zea, Barlocci & Markvardsen**
**Plaza Catalunya, 1**
**08002 Barcelona (ES)**

(54) **SYSTEM AND APPARATUS FOR THE NON-INVASIVE MEASUREMENT OF BLOOD PRESSURE**

(57) The present invention relates to a system for the estimation of the systolic (SBP), diastolic (DBP) and average (MAP) blood pressure. Said system establishes a physiological model of the pulse wave combined with its energy for, afterwards, generating a fixed length vector containing the previous model's values with other variables related to the user like, for example, age, sex, height, weight, etc... This fixed length vector is used as an input of a function estimator system based on "random forests" for the calculation of the three variables of interest. The main advantage of this function estimator lies in that it does not impose any restriction beforehand over the function to be estimated, and it is also very reliable with heterogeneous data, as in the present invention's case.

FIG.2

**Description**

**FIELD OF THE INVENTION**

The present invention refers to a system for a non-invasive measurement of systolic, diastolic and average blood pressure, regardless of the Oscillometric and Korotkoff methods. To do so, a stochastic model of the physiology of the pressure pulse and its instant energy is developed, combined with a system to approximate functions based in 'random forests'. The input signal is the preprocessed version of the plethysmographic pulse combined with other patient's variables.

**BACKGROUND OF THE INVENTION**

The principal function of blood circulation is to satisfy the needs of the tissues (i.e. transporting nutrients to the tissues, taking away the waste products, transporting hormones and maintaining the correct balance of all the tissue's liquids).

The relationship between the blood flux control related to the tissue's needs, and the heart control and the blood pressure necessary for the blood flux are quite hard to understand, and there is a lot of literature within the field, also existing a lot patents about the hemodynamic administration of the patients which require hemodynamic control like, for example, critic or hyper-tense patients.

Since the heart pumps the blood to the aorta in a continuous way, the pressure of this artery is high (a mean of 100mmHg). Since the cardiac pump is based on pulse, as an average, the artery pressure fluctuates between the systolic (SBP) of 120mmHg and a diastolic pressure (DBP) of 80mmHg. When the blood flows through the systemic circulation, its average pressure (MAP) is reduced progressive until, approximately, 0mmHg in the moment when it reaches the ending of the vena cava in the right auricle of the heart.

The pressure in the systemic capillary varies from 35mmHg, near the arterial extremes, to such low levels as 10mmHg, near the vein extremes, but its average functional pressure in most of the vascular bed is of about 17mmHg, an enough low pressure so that few plasma quantity can go through the porous capillary while allowing the diffusion of nutrients to the tissue cells.

Regarding the lung circulation, its pressure is also pulsed, as in the aorta, but with a systolic pressure of 25mmHg and a diastolic pressure of 8mmHg with an average lung arterial pressure of only 16mmHg. The lung capillary pressure is only 7mmHg. Nevertheless, the total blood flux going through the lung per minute is the same as in the systemic circulation. These low pressures in the lung system are adequate to the lung's needs, since the capillary only require the exposure to blood for the exchange of gases and the distances the blood has to go through before returning to the heart are low. Based on this, it can be concluded that the respiratory function and the gas exchange are very important in the hemodynamics of the patients and, therefore, their blood pressures.

Specifically, three basic principles exist within the circulatory function:

1. Blood flux of all the body's tissues is controlled based on the needs of the tissues, since when the tissues are active, they require more blood flux than when in standby (i.e. metabolic function).

2. The control of the cardiac expense (CE) is controlled by the sum of all the local tissue fluxes (i.e. the vein return whose response is the pumping back to the arteries from which it comes, done by the heart). In this sense, the heart acts as a state machine in response to the needs of the tissues. However, the heart's response is not perfect and it needs special nervous signals which make it pump the necessary blood quantities.

3. The blood pressure is controlled independently by means of a local blood flux control and/or by the control of the cardiac expense. For example, when the pressure decreases below its normal average value (100mmHg) a cascade of reflect impulses is produced which results in a series of circulatory changes to reestablish said pressure to its normal value. Said nervous signals increase the pumping pressure of the heart, the contraction of the big venous reservoirs to give more blood to the heart with a generalized contraction of much of the arterioles of all the body, in such a way that it accumulates in the arterial tree.

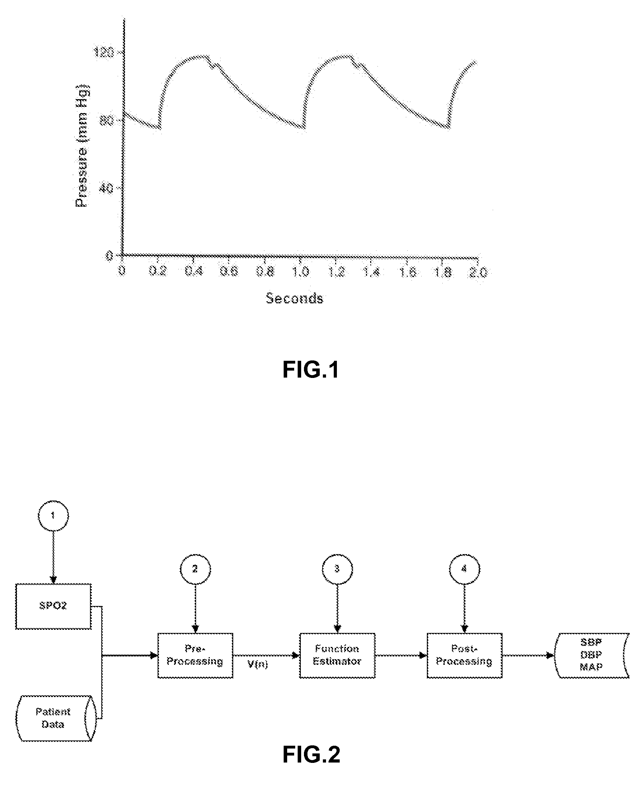

With each new heart beat, a new wave of blood fills the arteries. Because of the distensibility of the artery system, the blood flows both while the cardiac systole and diastole. Also, in normal conditions, the capacity of the arterial tree decreases the pressure of the pulses such that they almost disappear when the blood arrives to the capillaries, thus guaranteeing an almost continuous blood flux (with very few oscillations) in the tissues. Figure 1 shows a typical register of the pressure pulses obtained in an invasive form by means of a catheter in the aorta's root. To a young normal adult, the pressure during the cardiac systole (SBP) is approximately 120mmHg while during the diastole (DBP) is approximately 80mmHg. The difference between both pressures is called the pulse pressure (PP) and, in normal conditions, is approximately 40mmHg.

Two principal factors affect the pulse pressure:

1. The heart's systolic volume.
2. Total distensibility (capacity) of the arterial tree.

In general, a higher systolic volume means a higher quantity of blood that has to fill the arterial tree with each heart beat, with a higher increase and decrease of the pressure during the systole and diastole, which leads to a higher PP.

On the other hand, the PP may also be defined as the proportion between the systolic volume and the capacity of the arterial tree. Any process of circulation affecting any of these two factors will also affect the PP.

Based on the previously described, when the heart pumps blood to the aorta during the systole, in the beginning of the pumping, only the proximal portion of said artery distends since the blood's inertia does not allow its fast movement to the outlying vessels. However, the increase of the pressure on the central aorta surpasses rapidly said inertia and the wavefront of the distention extends through all of the aorta. This phenomenon is known as the pressure pulse transmission in the arteries. While said wavefront spreads through the arterial tree, the contours of the pressure pulse will attenuate during its transmission to the outlying vessels (absorption of the pressure pulses).

To measure the arterial pressure in the human being it is not reasonable to use an invasive catheter in a principal blood way, as previously described, except in critical cases (patients with an hemodynamic severe compromise like, for example, patients with a septic shock or multi-organic failure). Instead, the determining/monitoring of the arterial pressure is done by means of indirect methods such as the auscultation method (Korotkoff sounds). In this method, a stethoscope is displayed in the antecubital artery and a hose is inflated of arterial pressure along the higher part of the arm. While the hose compresses the arm with such a low pressure that the artery remains distended by the blood, no sounds are heard through the stethoscope, although blood circulates along the artery. However, when the hose pressure is high enough as to occlude the artery during part of the cycle of the arterial pressure, a sound is heard with each pulse. Therefore, in this method, first the hose pressure is increased way above the SBP in such a way that while this pressure is higher than the SBP of the braquial artery, no sound will be heard. In this moment the hose pressure is started to be decreased and, just in the instant when the pressure falls below the SBP pressure, the blood starts to flow through the artery and the Korotkoff sounds are heard synchronous to the cardiac beat. In this moment, the SBP is determined. While continuing decreasing the hose pressure, the quality of the Korotkoff changes with a rhythmic and rough sound. In the moment when the hose pressure equals the DBP said sounds stop being heard and said pressure is thus determined. Said system is considered as a reference within the medical community and literature and patents exist about it, which describe systems and electronic apparatus for determining the arterial pressure by this method.

A complementary system exists based on the measurement of the oscillations of a fluid column (normally mercury) caused by the propagation of the pressure wave. Said system is known as an oscillometric method. Literature also exist describing said method and several patents which describe systems and apparatus for determining the arterial pressure by means of said method. Said methods may be combined to improve the determination of arterial pressure. Summarizing, the previously described methods are based on mechanical methods, which compare pressures in a physical form.

The transmission of the pressure pulse is closely related with the photo-plethysmographic pulses (PPG) since these devices measure the changes in the light absorption, normally for wavelengths near infra-red (NIR), of the blood's hemoglobin and the obtained signal is proportional to the pressure pulse. In fact, the PPG may be considered as a low cost technique for measuring the changes in the blood's volume in a micro vascular level (normally a finger or ear lobe), being used in a non-invasive form on the skin of the patient. Said technology is implemented on commercial medical devices such as, for example, digital pulsioximeters and vascular diagnosis systems (for example, with PPG arrhythmias or extra-systoles may be detected in a reliable way).

Several patents exist referred to the use of the PPG, obtained by several means, for indirectly estimating the arterial tension.

Patent application US19740523196 establishes a system for the continuous monitoring of the SBP from the differentiation of the PPG signal in the beginning of the cardiac diastole.

Patent application US4418700 describes a system for estimating the SBP and DBP from the blood's volume, cardiac expense, etc.. represented by means of an analytic model and deterministic of the circulatory system. Said system requires a specific calibration for each patient which is reflected in the mathematical models found in said invention (K constant).

Patent application US4030485 describes a method for continuously monitoring the DBP based on the time lapses between peaks of the electrocardiographic signal (ECG) and the pulses detected by means of a pulse detector. Said invention is based on the fundamental principle that the transmission time of the pulses varies with the arterial pressure. The measurement system of the SBP is initially calibrated in a specifically for each patient with a sphygmomanometer (mechanical method).

Patent application US5140990 describes the method for continuously monitoring the SBP and DBP based on the PPG signal. The SBP and the DBP are determined from the blood volume obtained with the PPG and the SBP and DBP measures during the calibration period specific for each patient using a constant parameter K related to the arterial pressure - blood volume of the patient, which is determined before the average value.

Patent application US5237997 describes the method for continuously measuring the medium arterial pressure (MAP) from the transit time of the pulses of the PPG signal in the ear's lobe. The SBP and the DBP are obtained from measuring the density of the blood volume in said lobe. Said invention requires a calibration of the individual arterial tension values by conventional means (oscillometric or Korotkoff).

Patent applications US5865755 and US5857975 describe a method for determining the SBP and DBP from the ECG and PPG signals. The arterial pressure is obtained from the arrival times of the pulses, the shape of the volumetric wave and the cardiac rhythm for each pulse. Said patents use the time differences between the R waves of the ECG and the start of the PPG pulse with the difference of times between the start of the PPG pulse and the 50% of the amplitude, for determining the arterial pressure.

Patent application describes a system and apparatus for measuring the arterial tension from the transit time of the pulses and, at least, the cardiac rhythm and the pulse area after calibrating with a conventional system and a linear regressive analysis.

European patent application EP0443267AI 1 describes the technique for establishing a system NIBP based on two PPG sensor displayed in different parts of the body and calculating the difference between transit times of the average pulses with said sensors, to determine changes in the blood volume pumped by the heart. Said system requires a calibration by means of a conventional system.

Patent US2004/0260186A1 describes a system for obtaining different physiological parameters from the PPG. More specifically, said patent performs an estimation of the respiratory rhythm, cardiac rhythm variability of the cardiac rhythm, variability of the blood volume, information on the autonomous nervous system and monitoring the relative changes (not absolute ones) in the arterial pressure.

Patent US2006/0074322A1 describes a system for measuring the arterial tension without a hose, based on the photoplethysmography (PPG) principle. Said patent, although it claims a system and apparatus for the measurement of the arterial tension without a hose, it requires the calibration for each user based on the oscillometric and Korotkoff principles. Once the system is calibrated, this can be used exclusively and in a personalized way by means of the PPG principle.

Patent US2007/0032732A1 describes a system and apparatus for obtaining the blood volume found in the arterial tree by means of a harmonic analysis of the shape of the cardiovascular wave (pressure pulse obtained from the PPG) for the obtaining of the fundamental frequencies of the PPG wave and to obtain from them, the blood volume.

Patent US2008/0045846A1 describes a system for monitoring in a non-invasive way the arterial tension (NIBP) including an inflatable hose and a photoplethysmograph implemented in a pulse oximeter (SpO2) for determining the initial inflating pressure of said hose. The fundamental principle of said invention is in that from an inflating pressure of the hose higher than the SBP, the PPG wave pulses disappear. This way, the user is protected from an over-inflation of the hose and other safer measurements can be performed.

Patent US2008/0082006A1, contrary to the above one, describes a NIBP system which uses the PPG signal to reduce and/or optimize the blood pressure measurement time. In said invention, the hose's de-inflating period is controlled by means of the PPG signal.

Although a lot of patents are found which use the PPG signal as a basic functioning principle, either combined with an individual calibration or with its group of blood pressure conventional systems, it's still to be resolved the need to find a safer, reliable continuous monitoring system, which does not include mobile mechanical components, being non-invasive for the clinical determination of arterial pressure (NIBP), which does not require an individualized calibration by means of a sphygmomanometer and which can function without support of any conventional means (or any evolution of the oscillometric and Korotkoff methods previously described) for its correct performance and operation.

**BRIEF SUMMARY OF THE INVENTION**

According to the prior art of the invention previously described, the pulse suffers an attenuation and alteration of its morphology, which depends on the blood pressure. This effect varies depending on the difference between the SBP and the DBP. The proposed system and apparatus of the present invention is based in the interference of the functional relationship between the shape of the pulse (PPG) and the pressure levels where the information is deduced from the dependence between the pulse and its statistics with the blood pressure state of the patient.

The input information to perform the estimation of SBP, DBP and MAP is processed to ease the job of the function estimator. Since the the PPG signal has a variable duration, a treatment is performed to generate a fixed length vector for each measurement. This vector contains information related to the pulse (auto-regression coefficients and mobile mean), the average distance between pulses, its variance, information related to the instant energy, energetic variability and clinical information of the person like, for example, sex, age, weight, height, clinical information of the patient (body mass index or similar measurements), etc...

The system for the function's interference works blindly in the sense that no functional restriction is imposed to the relation between pulse and blood pressure levels. Since the functional form which related the PPG with the blood pressure levels is unknown, a system to infer said function has been chosen which is reliable in front of irrelevant input variables like clinical information and parameters derived from the waveform of the PPG. Also, said technique is related with other parameters as it has been discussed in the prior art of the present invention. The preferred system for the estimation of functions of the present invention is the "random forests" in comparison to other "machine-learning" systems and pattern recognition like, for example, decision and regression trees (CART), Splines, classifiers committees, Support Vector Machines and Neural networks. The random forests are based on the parallel generation of a plurality of decision trees, which estimate a function with a selection of random variables in each node, the pruning of the nodes not being performed, and each tree being trained with a random sub-set of the training database, in such a way that each tree presents a different systematic generalization error. Therefore, when performing an average of each tree's estimations, the systematic errors are compensated and the estimation variance decreases.

The implementation of the present invention comprises two different steps. The first step is the training of the system, which is performed only once and, therefore, does not require any later calibration/personalization. This step consists of the obtaining of a database with information about different parameters of patients including, sex, weight, age, etc... and a recording of the plethysmographic wave. This information is used in the estimation of the parameters of the decision trees and are stored within the system.

The second step consists of loading the information of the set of trees obtained in the training step and recording the plethysmographic wave of the patient in the moment of the measurement with other variables such as, for example, sex, weight, age, etc... In this step, the system reads the information of the plethysmographic pulse, performs the processing of the same and generates a fixed length vector with the information describing the signal. An additional information is added to this vector, regarding the person, and a set of "random forests" is applied, which calculate several intermediate functions of the variables of interest. Later, the variables of interest are calculated from said intermediate functions.

## BRIEF DESCRIPTION OF THE DRAWINGS

Figure 1 of the present invention shows the shape of the pulse wave obtained by means of an invasive catheter.

Figure 2 of the present invention shows a general block diagram of the described system and apparatus.

Figure 3 of the present invention shows the shape of a plethysmographic wave obtained by means of a digital pulse oximeter.

Figure 4 shows a detailed block diagram of the pre-processing system described in the present invention.

Figure 5 shows the detailed block diagram of the AR filter for the establishment of the stochastic model of the physiology of the pressure pulse described in the present invention.

## DETAILED DESCRIPTION OF THE INVENTION

The present invention consists of a system for the continuous monitoring of the blood pressure (systolic, diastolic and average) (figure 2) whose data are evaluated by means of a plethysmographic signal capturing device (1) (optic, acoustic or mechanical signals) the preferred implementation of the invention consisting of a pulse oximeter system (SpO2). The PPG information is combined with other data of the person such as, for example, age, sex, height, weight, etc... and it is linked with a digital pre-processing system (2), which implements a stochastic model with the physiology of the circulatory system presented in the prior art. Said system captures in a better way all the parameters affecting in a random way the transmission of the pressure pulse and, therefore, the blood pressure.

The vector of the obtained stochastic model is, also, linked to a digital system (3) which approximates functions based on the "random forests" whose main function is to estimate the basic parameters (SBP, DBP and MAP) with other different functions related with these, to decrease the estimation error in the step of post-processing (4). The main function of the system (4) is to estimate the final values of the SBP, DBP and MAP by means of an average of the functions of the previous step (3) to decrease the systematic error (bias) and the variance of the obtained SBP, DBP and MAP estimations. The systems (2, 3, and 4) are implemented by means of a CPU comprising several devices such as FPGA, DSP or microcontrollers.

The system (1) for obtaining the PPG curve implements a non invasive, low cost and simple technique, for detecting the changes in the volume of the microvascular net of a tissue. The most basic implementation of said system requires of few opto-electronic components including:

> 1. One or several sources for the illumination of the tissue (for example, the skin)
> 2. One or more photo-detectors for the measurement of small variations on the light intensity associated with the changes in the infusion of the tissue in the detection volume.

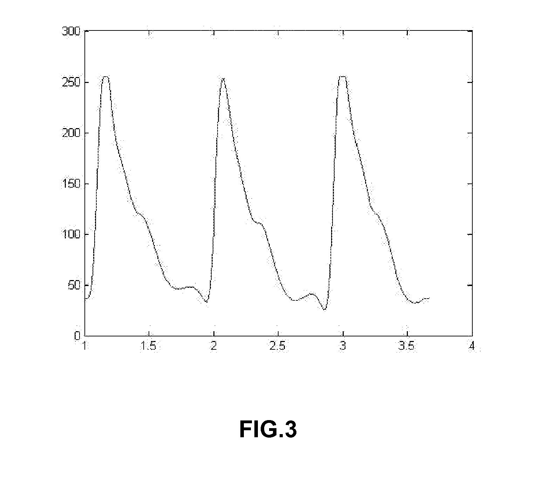

The PPG is normally used in a non-invasive way and operates in the infra-red or near infra-red (NIR) wavelengths. The most recognized wave form with the PPG is the peripheral pulse (figure 3) and it is synchronized with each heart beat. It is important to acknowledge the similarity between the waves obtained by means of the PPG and the pulses obtained by means of an invasive catheter (figures 1 and 3). Because of the highly valued information obtained by means of the PPG, it is considered as a main input of the present invention.

The PPG wave comprises a physiological pulsed wave (AC component) related to the changes in the blood volume synchronized with each heart beat. Said component is superimposed to another basal low frequency component (DC component) related to the respiratory rhythm, the activity of the central nervous system and the thermo-regulation. The fundamental frequency of the AC component is found around 1Hz depending on the cardiac rhythm (figure 3).

The interaction between the light and the biological tissues is complex and includes optical processes like the scattering, absorption, reflection, transmission and fluorescence. The selected wavelength for the system (1) is very important because of the following:

> 1. Water optical window: the main component of tissues is water. This highly absorbs the ultraviolet wavelengths and the long wavelengths within the infra-red band. A window exists in the water absorption spectrum which allows the visible light (red) or NIR to pass through the tissue allowing the measurement of the blood flux or its volume in these wavelengths. Therefore, the present invention will use NIR wavelengths for the system (1).

> 2. Isosbestic wavelength: significant differences exist on the absorption between the oxi-hemoglobin (HbO2) and the reduced hemoglobin (Hb) except for this wavelength. Therefore, the measurements performed on this wavelength (i.e. near the 805nm, for the NIR range) the signal won't be affected by the changes in the oxygen saturation of the tissue.

> 3. Penetration in the tissue: the deep of the light penetration in a tissue for a determined radiation intensity is also a function of the selected wavelength. For the PPG, the penetration volume (depending on the probes used) is of approximately 1 cm3 for transmission systems like the one used in (1).

The PPG pulse (figure 3) presents two different steps: the anacrotic step, which represents the rise of the pulse, and the catacrotic step, which represents the fall of the pulse. The first step is related with the cardiac systole while the second is related with the diastole and the reflections suffered by the wave in the periphery of the circulatory system. A dicrotic pulse in the catacrotic step is also usually found in the PPG, in healthy patients without arteriosclerosis or arterial hardening.

As it has been described in the prior art of the invention, the propagation of the pressure pulse PP along the circulatory tree has to be taken into account. Said PP changes its shape while it moves towards the periphery of the circulatory tree, being amplified/attenuated and suffering alterations of its shape and temporal characteristics. These changes are because of the reflections of the PP which are caused by the narrowing of the arteries in the periphery. The propagation of the PP pulse is further affected by a phase distortion frequency dependant.

Because of the physiological process, which generates the pulse, the ARMA models (Auto-regressive mobile mean model), characterize the generating mechanism and therefore, this models have been considered as a representation of the PP. To model in parallel the non-linear interactions, the Teager-Kaiser operator is used, coupled with an AR (Auto-regressive) system (2).

As seen in figures 1 and 3, the PP is similar to the PPG, and similar changes are observed during vascular pathologies (cushioning caused by stenosis or a pulsatility change).

The pulsi-oximeter of the system (1) uses the PPG to obtain information about the oxygen saturation (SpO2) in the arteries of the patient. As previously described, the SpO2 may be obtained by means of tissue illumination (normally the finger or the ear lobe) in the red and NIR wavelengths. Normally, the SpO2 devices use the commutation between both wavelengths to determine said parameter. The amplitudes of both wavelengths are sensitive to the changes in SpO2 because of the absorption difference between the HbO2 and Hb for these wavelengths. The SpO2 may be obtained from the ratio between the amplitudes, the PPG and the AC and DC components.

In pulsi-oximetry, the light intensity (T) transmitted through the tissue is commonly known as DC signal and it is a function of the optical properties of the tissue (i.e. absorption coefficient $\mu_a$ and scattering coefficient $\mu_s$). The arterial pulsation produces periodical variation in the oxi and deoxi hemoglobin concentrations, further resulting in periodical variations of the absorption coefficient.

The intensity variations of the AC component of the PPG may be the following:

$$AC = \Delta T = \frac{\partial T}{\partial \mu_a}\Big|_{\mu_a,\mu_s'} \Delta \mu_a \quad (I).$$

This shape of the physiological wave is proportional to the variation of the light intensity, which it is itself a function of the scattering and absorption coefficients ($\mu_a$ y $\mu'_s$ respectively). The $\Delta \mu_a$ variations may be defined as a linear variation of the oxi and deoxi hemoglobin variations ($\Delta c_{ox}$ and $\Delta c_{deox}$):

$$\Delta \mu_a = \varepsilon_{ox} \Delta c_{ox} + \varepsilon_{deox} \Delta c_{deox} \quad (II),$$

Being $\varepsilon_{ox}$ and $\varepsilon_{deox}$ the extinction efficient (i.e. light fraction lost because of the scattering and absorption by distance unit in a determined environment) of the oxi and deoxi hemoglobin. Based on the previous equations, the arterial oxygen saturation ($SpO_2$) may be defined by:

$$SpO_2 = \frac{\Delta c_{ox}}{\Delta c_{ox} + \Delta c_{deox}} \quad (III).$$

The expression of the $SpO_2$ depending on the AC component may be obtained by means of the direct application of equations (I) and (III) to the selected wavelengths (red and NIR):

$$SpO_2 = \frac{1}{1 - \dfrac{x\varepsilon_{ox}(NIR) - \varepsilon_{ox}(R)}{x\varepsilon_{deox}(NIR) - \varepsilon_{deox}(R)}} \quad (IV).$$

Wherein,

$$x = \frac{\dfrac{\partial T(NIR)}{\partial \mu_a}\Big|_{\mu_a,\mu_s'}}{\dfrac{\partial T(R)}{\partial \mu_a}\Big|_{\mu_a,\mu_s'}} \frac{AC(R)}{AC(NIR)} \quad (V).$$

Normalizing the AC component with the DC component to compensate the effects on a low frequency not related with the synchronous changes in the blood (see prior art), the following is obtained:

$$R = \frac{\dfrac{AC(R)}{DC(R)}}{\dfrac{AC(NIR)}{DC(NIR)}} \, .$$

Including this parameter in (IV) the following is obtained:

$$SpO_2 = \frac{1}{1 - \dfrac{kR\varepsilon_{ox}(NIR) - \varepsilon_{ox}(R)}{kR\varepsilon_{deox}(NIR) - \varepsilon_{deox}(R)}} \; (VI).$$

Being

$$k = \frac{\dfrac{\Delta T(NIR)}{DC(NIR)}}{\dfrac{\Delta T(R)}{DC(R)}} \, .$$

Wherein $\Delta T(NIR)$ and $\Delta T(R)$ correspond to equation (I) evaluated in the R and NIR wavelengths.

Although equation (VI) is an exact solution to the SpO2, k cannot be evaluated since it does not have $T(\mu_a, \mu_s')$. Anyway, k and R are functions of the optical properties of the tissue, being possible to express k as a function of R. More particularly, it is possible to express k as a linear regression of the following:

$$k = aR + b \quad (VII).$$

This linear regression implies a calibrating factor empirically derived but assuming a flat wave with P intensity, its absorption coefficient is defined as follows:

$$dP = \mu_a P dz \quad (VIII),$$

Wherein dP represents the differential change of the intensity of a light ray going through an infinitesimal dz in a homogeneous environment with an absorption coefiicient $\mu_a$. Therefore, integrating on z, the Beer-Lambert law is obtained:

$$P = P_0 e^{\mu_a Z} \quad (IX),$$

Assuming that $T \approx P$ the equation (VII) is reduced to k=1, which is the preferred approximation in the pulsi oximetry measurement performed in the present invention.

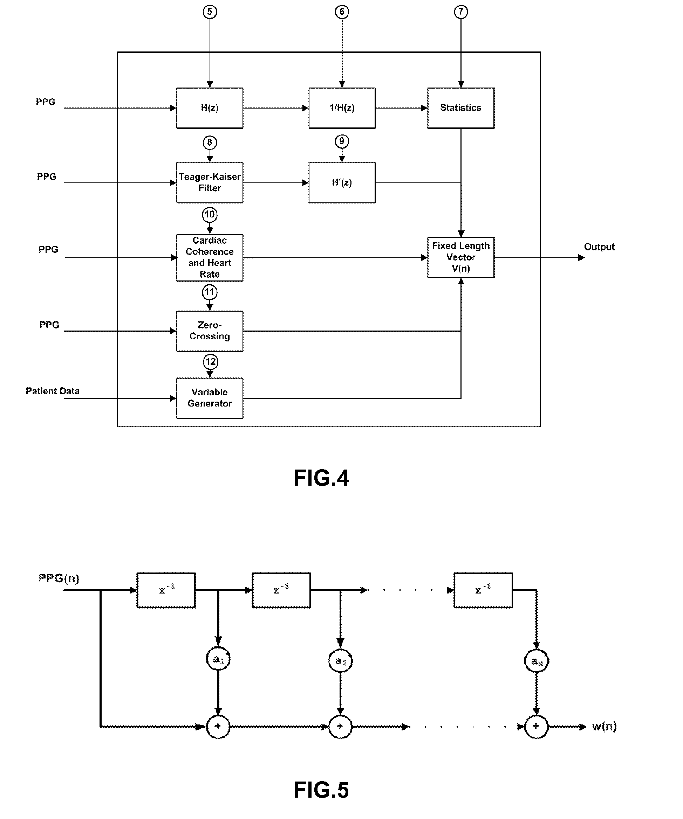

The obtained PPG signal of the system (1) is used as an excitation of the system (2) (figure 4) of the present invention, whose main function is to establish a stochastic model of the circulatory function for the estimation of SBP, DBP and MAP.

In the prior art of the present invention, different parameters have been described, which have an important role in both the shape and the propagation of the pressure pulse PP. Said parameters are related to the cardiac expense, cardiac rhythm, cardiac synchronization, respiratory rhythm, metabolic function, etc... It has also been previously detailed the intimate relationship between PP and PPG. Therefore, since the previously detailed parameters have a key role in the type of propagation of the PP, it is assumed that they will also affect the PPG.

Taking into account this, the preferred implementation of the present invention uses a system of stochastic modeling ARMA (q,p) (Auto-regressive mobile mean model with a q of approximately of q (MA) and p (AR)) (5).

The temporal series PPG(n), PPG(n-1),..., PPG(n-M) may be modeled as an AR process of a p=M order if the following equation in finite differences is satisfied

$$PPG(n) + a_1 PPG(n-1) + \cdots + a_M PPG(n-M) = w(n) \qquad (X)$$

Wherein the coefficients $[a_1, a_2, \cdots, a_M]$ are the parameters known as AR and w(n) is a white process. The term $a_k PPG(n-k)$ is the interior product of the coefficient $a_k$ and PPG(n-k), wherein k=1,..,M. Equation (X) may be re-written like:

$$PPG(n) = v_1 PPG(n-1) + v_2 PPG(n-2) + \cdots + v_M PPG(n-M) + w(n) \qquad (XI),$$

wherein $v_k = -a_k$ .

From the previous equation, the actual value of the pulse PPG(n) is determined as being equal to a finite linear combination of previous values (PPG($n$ - $k$)) plus a prediction error term w(n) . Therefore, rewriting the equation (X) as a linear convolution, the following is obtained:

$$\sum_{k=0}^{M} a_k PPG(n-k) = w(n) \qquad (XII).$$

Without losing generality, $a_0= 1$ can be defined as the Z transformation of the predictor filter which will be defined by:

$$A(z) = \sum_{n=0}^{M} a_n z^{-n} \qquad (XIII).$$

Defining PPG(z) as the Z transformation of the PPG pulse, then:

$$A(z)PPG(z) = W(z) \qquad (XIV),$$

wherein

$$W(z) = \sum_{n=0}^{M} v(n) z^{-n} \qquad (XV).$$

Figure 5 shows the analysis filter of the AR component of the pulse PPG($n$) obtained in the system (1).

Regarding the MA (Mobile Average) with a q=K order of the pulse PPG($n$) this can be described as the response of a discrete linear pulse excited by a Gaussian white noise. Therefore, the MA response of said filter written as EDF will be:

$$PPG_{MA}(n) = e(n) + b_1 e(n-1) + \cdots + b_K e(n-K) \qquad (XVI),$$

wherein $[b_1, b_2,...,b_K]$ are the constants known as MA parameters and $e(n)$ is a white process with a null mean and variance $\sigma^2$. Therefore, relating equations (XII) and (XVI) the following is obtained:

$$PPG(n) = e(n) + \sum_{k=0}^{p} a_k PPG(n-k) + \sum_{k=0}^{q} b_k e(n-k) \qquad \text{(XVII)},$$

being $e(n)$ the error terms of the ARMA(q,p) model. Performing a Z transformation in (XVII) the following is obtained:

$$PPG(z) = \frac{B(z)}{A(z)} E(z) \qquad \text{(XVIII)},$$

since the first terms of the AR and MA vectors may be equaled to 1 without generality loss, the ARMA(q,p) filter expression (5) in the system (2) will be defined by:

$$H(z) = \frac{B(z)}{A(z)} \qquad \text{(XIX)}.$$

Being A(z) and B(z) the AR and MA components of PPG(n) respectively. The preferred embodiment of the present invention uses an ARMA model with a q=1 and p=5 order, although any p and q order may be used, comprised between [4,12].

Once the ARMA(q,p) model is calculated by means of Wold decomposition and the Levinson-Durbin recursion, the H(z) is generated and the input signal is filtered with the inverse of H(z) (6). Also, the residue statistics $e(n)$ are calculated with the sub-system (7). The obtained information of these subsystems is stored in the output fixed length vector $v(n)$.

The pre-processing system (2) of the present invention further comprises a sub-system (8) which calculates the Teager-Kaiser operator and models the output of it by means of an AR process with a p order equivalent to the previously described.

In this case, without a generality loss, the PPG pulsed is considered as an AM-FM modulated signal (both modulated in amplitude and frequency) of the type:

$$PPG(t) = a(t)\cos \int_{0}^{t} w(\tau(\tau) \qquad \text{(XX)}$$

Being $a(t)$ and $w(t)$ the instantaneous amplitude and frequency of the PPG. The Teager-Kaiser operator of a determined signal is defined by:

$$\Psi[x(t)] = [x'(t)]^2 - x(t)x''(t) \qquad \text{(XXI)}.$$

Being $x'(t) = \dfrac{dx(t)}{dt}$.

This operator applied to an AM-FM modulated signal of the equation (XX) results in the instant energy of the source producing the oscillation of the PPG. This is, $\Psi[PPG(t)] \approx a^2(t)w^2(t)$ (XXII), wherein the approximation error is not significant if the instantaneous amplitude $a(t)$ and the instantaneous frequency $w(t)$ are not varied too fast compared to the average value of $w(t)$; which is the PPG pulse case.

The AR process of p order of $\Psi[x(t)]$ is implemented with a filter (9) equivalent to the one of figure 5. The preferred embodiment of the present invention uses an AR model of p=5 order, although any other may be used with a p and q order between 4 and 12.

Once the stochastic models based on an ARMA (q,p) model (5, 6 and 7) and the ARMA(q,p) model are calculated with the Teager-Kaiser (8 and 9) operator, the present invention calculates the cardiac rhythm (HR) and the cardiac synchronization (i.e. the variability of the cardiac rhythm) from the PPG by means of a sub-system (10). The preferred embodiment of the present invention calculates the cardiac rhythm over temporal windows of the PPG which may vary between 2 seconds and 5 minutes with the autocorrelation function of the signal.

The pre-processing system (2) further comprises a sub-system (11), which calculates the zero passes of the PPG signal with the variance of these zero-passes. The preferred embodiment of the present invention calculates the cardiac rhythm over temporal windows of the PPG which may vary between 2 seconds and 5 minutes.

Finally, the pre-processing system (2) comprises a sub-system (12) for the generation of variables related with the patient, said variables being the following among others:

1. Sex, age, weight, height, if the patient has eaten any food, time of the day.
2. Body mass index.
3. Weight divided by age.
4. Weight divided by HR.
5. Height divided by HR.
6. HR divided by age.
7. Height divided by age.
8. Age divided by the body mass index.
9. HR divided by the body mass index.

All the obtained data in the subsystems which comprise the system (2) are stored in the fixed length output vector *V(n).*

Once the features fixed length vector *V(n)* is obtained, an estimation of the SBP, DBP and MAP may be performed by the system for approximating functions (3) based on 'random forests'. The function estimating system presented in this invention does not require any calibration once the "random forest" has been correctly trained.

More specifically, a "random forest" is a classifier which consist of a set of classifiers with a tree structure {*h (V,$\Theta_k$),*k =1*,* } wherein $\Theta_k$ are random independent vectors and identically distributed (i.i.d) wherein each vector places a vote for the most popular class of the input V. This approximation has a clear advantage in reliability compared to other classifiers based in a unique tree, and it does not impose any functional restriction on the relationship between the pulse and the blood pressure levels.

The "random forests" used in the present invention are generated by means of the growth of decision trees depending on the random vector $\Theta$ in such a way that the predictor *h*(V,$\Theta$) has numerical values. This random vector $\Theta$ associated with each tree results in a random distribution of each node and, at the same time, it also provides information about the random sampling of the training base, giving as a result different sub-sets of data for each tree. Based on this result, the generalization error of the classifier used in the present invention is defined by:

$$\mathrm{PE} = E_{V,Y}(Y - h(V))^2 \quad \text{(XXIII).}$$

Since the generalization error of the "random forest" is less than the one by a unique decision tree, defining:

$$\mathrm{Y} - \mathrm{h}(\mathrm{V},\Theta) \qquad \text{(XXIV),}$$

$$\mathrm{Y} - \mathrm{h}(\mathrm{V},\Theta') $$

the following is obtained:

$$\mathrm{PE}(\text{forest}) \le \rho\mathrm{PE}(\text{tree}) \qquad \text{(XXV).}$$

Each tree presents a different generalization error and p represents the correlation between the residues defined in (XXIV). This fact, implies that a minor relationship between residues (XXIV) results in major estimations. In the present invention, this minimum correlation comes from the random sampling process of the features vector of each node of the tree which is being trained in the subsystem (2). With the aim of decreasing even more the generalization error, the present invention estimates both the interest parameters (SBP, DBP and MAP) and their linear combinations.

The "random forests" consist of a set of decision trees of CART type ("Classification and Regression Trees), altered to introduce systematic errors (XXV) in each one and after, by means of a bootstrap system, a symmetric variability (both random processes are modeled by the parameter Θ in the analysis of the predictor $h(V,\Theta)$). The different systematic error in each embodiment is introduced by two mechanisms:

1. Random election in each node of a subset of attributes, making that an equivalence cannot be established on a statistic level of the partitions made in different trees between similar nodes, in such a way that each tree behaves in a different way.
2. Letting the trees grow to their maximum. In this case the trees act in a similar way as in a search table based on rules. Because of the sampling of the attributes, they are search tables with a different structure.

The result of this process is that each tree will present a different systematic error.

Furthermore, for each of these two modifications, each tree trains with a bootstrap type sample (i.e. a sample is taken from the input data, which leads to a part of the input data missing, while the other part is repeated). This bootstrap effect introduces a variability, which is compensated when making average estimations.

The global result of these features is a system (4), wherein the systematic error and the error variability can be easily compensated resulting more precise than other type of function estimators (XXV). In this system, the basic classifier is a tree, which decides based on levels, what is strong with input distributions with outliers or heterogeneous type data (like in the case of the present invention).

The preferred embodiment of the system (4) consist of obtaining random samples of two elements of 47 in a node level (being able to choose a level between 2 and 47) and a bootstrap size of 100, being possible to vary the size between 25 and 500.

The hand device according to the invention may comprise a screen to visualize data and control instructions for the functioning of the apparatus. It comprises at least an acoustic, mechanical and/or optic probe whose signals are interpreted by a post-processing system by means of a CPU implemented by means of a DSP, FPGA or microcontrollers. It also comprises work memories to store the data and operative processes of the system.

The invention also for-sees the manual device to comprise buttons or a switch panel, according to the state of the art, for activating and controlling the device, and batteries and/or access to an external power source.

Finally, the obtained results by means of the present invention may be transmitted to a PC to be analyzed by means of a serial port or a USB or network connection, for example, by means of WiFi or Bluetooth.

It is understood that alternatives with minor detail changes are comprised within the scope of the invention as described herein.

## Claims

1. System and apparatus for a non-invasive measurement of blood pressure, **characterized in that**:

   - they incorporate an stochastic model of the autoregressive mobile average type (ARMA) circulatory function (5), (6) and (7) on the input signal.
   - they incorporate a stochastic model of the autoregressive mobile average type (ARMA) blood pressure pulse on the Teager-Kaiser operator of the input signal (8) and (9).
   - they incorporate clinical data (sex, age, height, body mass index, etc.) and its functions.
   - they incorporate a function estimation system based on 'random forests' (3).

2. System and apparatus for a non-invasive measurement of blood pressure, according to the previous claim, **characterized in that** the input of the function estimation system (3) consists of a vector of a fixed size including the previous models and information of the patient (sex, age, height, weight, body mass index, pulse rhythm, cardiac coherence, zero-passes of the pre-processed input signal and variability of the zero-passes of the pre-processed input signal etc.).

3. System and apparatus for a non-invasive measurement of blood pressure, according to the previous claims, **characterized in that** the input signal of the system is a preprocessed plethysmographic wave optically, mechanically or acoustically obtained.

**4.** System and apparatus for a non-invasive measurement of blood pressure, according to the previous claims, **characterized in that** the plethysmographic wave has been obtained by means of a digital pulse oximeter.

**5.** System and apparatus for a non-invasive measurement of blood pressure, according to the previous claims, **characterized in that** the functions to be estimated in (3) are the basic parameters (SBP, DBP and MAP) and linear combinations of these parameters to decrease the estimation error in (3).

**6.** System and apparatus for a non-invasive measurement of blood pressure, according to the previous claims, **characterized in that** it incorporates a post-processing system (4), which performs the average of the system's (3) estimations to lower the systematic error and the variance of the estimated parameters (SBP, DBP and MAP).

**7.** System and apparatus for a non-invasive measurement of blood pressure, according to the previous claims, **characterized in that** the SBP, DBP and MAP estimation system is implemented by means of DSP devices, for example, by means of FPGA type microcontrollers.

**8.** System and apparatus for a non-invasive measurement of blood pressure, according to claim 1, **characterized in that** they comprise a manual device which incorporates at least an acoustic, mechanic and/or optic catheter, comprising inside a data processing system, by means of a CPU, aimed to reduced the variance of the estimated parameters (SBP, DBP, MAP).

**9.** System and apparatus for a non-invasive measurement of blood pressure, according to the previous claim, **characterized in that** said CPU is implemented by means of DSP, FPGA or microcontroller devices.

**10.** System and apparatus for a non-invasive measurement of blood pressure, according to claims 6 to 8, **characterized in that** they comprise a storing memory, for example a flash type memory.

**11.** System and apparatus for a non-invasive measurement of blood pressure, according to claims 6 to 9, **characterized in that** they comprise exterior connecting means to a PC, for example by means of a serial port, Bluetooth or USB (12), and/or network connecting means, for example by means of a WiFi, Zigbee or UWB.

**12.** System and apparatus for a non-invasive measurement of blood pressure, according claims 6 to 10, **characterized in that** they comprise a data visualizing screen.

**13.** System and apparatus for a non-invasive measurement of blood pressure, according to claims 6 to 11, **characterized in that** they comprise control switches, batteries and/or connection to an external power source.

**FIG.1**

**FIG.2**

**FIG.3**

**FIG.4**

**FIG.5**

# INTERNATIONAL SEARCH REPORT

| International application No. |
| --- |
| PCT/ ES 2009/000064 |

## A. CLASSIFICATION OF SUBJECT MATTER

**A61B 5/021** (2006.01)

According to International Patent Classification (IPC) or to both national classification and IPC

## B. FIELDS SEARCHED

Minimum documentation searched (classification system followed by classification symbols)

A61B5/02

Documentation searched other than minimum documentation to the extent that such documents are included in the fields searched

Electronic data base consulted during the international search (name of data base and, where practicable, search terms used)

INVENES,EPODOC,WPI,BIOSIS,MEDLINE,NPL,INSPEC

## C. DOCUMENTS CONSIDERED TO BE RELEVANT

| Category* | Citation of document, with indication, where appropriate, of the relevant passages | Relevant to claim No. |
| --- | --- | --- |
| A | Jingyu et al. "A novthe auto regression and fuzzy-neural combination method to identify cardiovascular dynamics", IEEE Proceedings of the World Automation Congress (IEEE Cat. No. 04EX832C), Piscataway,NJ,USA, vol.18, pgs 31-37 | 1 - 13 |
| A | Sheng Lu et al. "A new algorithm for liner and nonliner ARMA modthe parameter estimation using affine geometry", IEEE Transactions on biomedical Engineering, 2001, vol. 48, nº 10, Pages 1116-1124 | 1 - 13 |
| A | Chon K H et al. "Robust nonliner autoregressive moving average modthe parameter estimation using stochastic recurrent artificial neural networks". Proceedings of the first joint BMES/EMBS Conference Serving Humanity, Advancing Technology, October 13-16, '99, Atlanta, GA, USA | 1 - 13 |
| A | Di Virgilio et al. "A multivariate time-variant AR method for the analysis of heart rate and arterial blood pressure". Medical Enginnering & Physiscs, 1997, vol. 19, nº 2, pages 109-124 | 1 - 13 |

☒ Further documents are listed in the continuation of Box C.      ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 29 June 2009      (29.06.2009) | **(03/07/09)** |
| Name and mailing address of the ISA/ O.E.P.M. <br><br> Paseo de la Castellana, 75 28071 Madrid, España. <br> Facsimile No.   34 91 3495304 | Authorized officer <br><br> A. Cardenas Villar <br><br> Telephone No. +34 91 349 53 93 |

Form PCT/ISA/210 (second sheet) (July 2008)

## INTERNATIONAL SEARCH REPORT

| | International application No. |
|---|---|
| | PCT/ES 2009/000064 |

| C (continuation). | DOCUMENTS CONSIDERED TO BE RELEVANT | |
|---|---|---|
| Category* | Citation of documents, with indication, where appropriate, of the relevant passages | Relevant to claim No. |
| A | Chin-Te Chen et al. "Adaptive contol of arterial blood pressure with a learning controller based on multilayer neural networks". IEEE Transactions on Biomedical Engineering, 1997, vol. 44, n° 7, pages 601-609 | 1 - 13 |
| A | WO 0072750 A1 (Massachusets Institute of Technology) 07.12.2000 the whole document | 1 - 13 |
| A | DE 10033171 A1 (Elter, P. et al.) 17.01.2002 the whole document | 1 - 13 |
| A | US 2005261593 A1 (Zhang et al.) 24.11.2005 the whole document | 1 - 13 |
| A | US 6527725 B1 (COLIN CORP) 04.03.2003 the whole document | 1 - 13 |

Form PCT/ISA/210 (continuation of second sheet) (July 2008)

## INTERNATIONAL SEARCH REPORT
Information on patent family members

| International application No. |
| --- |
| PCT/ ES 2009/000064 |

| Patent document cited in the search report | Publication date | Patent family member(s) | Publication date |
| --- | --- | --- | --- |
| WO 0072750 A | 07.12.2000 | US 6413223 B<br>JP 2003500148 T | 02.07.2002<br>07.01.2003 |
| DE 10033171 A | 17.01.2002 | NONE | ------------ |
| US 2005261593 A | 24.11.2005 | CN 1698536 A<br>US 7479111 B | 23.11.2005<br>20.01.2009 |
| US 6527725 B | 04.03.2003 | NONE | ------------ |

**EP 2 343 008 A1**

## REFERENCES CITED IN THE DESCRIPTION

**Patent documents cited in the description**

- US 19740523196 A
- US 4418700 A
- US 4030485 A
- US 5140990 A
- US 5237997 A
- US 5865755 A
- US 5857975 A

- EP 0443267 A1
- US 20040260186 A1
- US 20060074322 A1
- US 20070032732 A1
- US 20080045846 A1
- US 20080082006 A1